Europäisches Patentamt

European Patent Office

Office européen des brevets

(11) Publication number: **0 266 957 B1**

(12) **EUROPEAN PATENT SPECIFICATION**

(45) Date of publication of patent specification: **30.12.92**  (51) Int. Cl.⁵: **A61M 29/02**

(21) Application number: **87309512.9**

(22) Date of filing: **28.10.87**

(54) Two balloons angiplasty catheter.

(30) Priority: **04.11.86 US 927239**

(43) Date of publication of application:
**11.05.88 Bulletin 88/19**

(45) Publication of the grant of the patent:
**30.12.92 Bulletin 92/53**

(84) Designated Contracting States:
**DE ES FR GB IT NL**

(56) References cited:
**EP-A- 0 063 859
EP-A- 0 261 831
US-A- 4 307 722
US-A- 4 423 725
US-A- 4 490 421**

(73) Proprietor: **C.R. BARD, INC.
731 Central Avenue
Murray Hill New Jersey 07974(US)**

(72) Inventor: **Crittenden, James Frederick
232 Worcester Road
Hollis New Hampshire 03049(US)**

(74) Representative: **Woodward, John Calvin et al
Venner Shipley & Co. 20 Little Britain
London EC1A 7DH(GB)**

## Description

This invention relates to improvements in balloon dilatation catheters for use in angioplasty procedures. Balloon angioplasty procedures have been used with increasing regularity in recent years as a treatment for stenosed arteries, such as the coronary arteries. The procedure involves the use of a special catheter such as that disclosed in US-A-4.307.722 on which the preamble of claim 1 for ES and IT is based which has a balloon at its distal end. The catheter is inserted percutaneously into the patient's arterial system and is advanced and manipulated to place the balloon within the stenosis in the coronary artery to be treated. The balloon then is inflated under substantial pressure to press the plague and plague laden arterial wall radially outwardly to enlarge the stenosed region of the artery. When successful, the procedure avoids the necessity for coronary artery bypass surgery.

During an angioplasty procedure, it is not uncommon for the physician to have to make a catheter exchange, to substitute another balloon catheter for the one in place in the patient's artery. The catheter exchange is a somewhat time-consuming manipulation. It involves removal of the guidewire from the catheter and insertion of a long exchange wire into the catheter, then removal of the catheter from the patient by withdrawing it over the exchange wire, then advancing the next catheter onto and along the exchange wire to advance it to the region of the stenosis and then removing the exchange wire. Typically, the exchange wire will be replaced by another shorter guidewire which may be used to facilitate subsequent manipulation and placement of the catheter in the stenosis.

The need for a catheter exchange may be prompted by several factors. Among the situations which requires a catheter exchange are those in which there is a need for a catheter with a different diameter balloon This may occur, for example, in treating a patient having multiple stenosis either in the same or in different arteries. Because of the nature or size of the stenosis or the size of the artery in which the stenoses is located, it may be desirable to use a catheter with a smaller or a larger diameter balloon than that which is in place in the patient's arteries. Typically, such difference in size of stenoses have required the use of different size balloon dilatation catheters. In recent years, as balloon angioplasty techniques have developed, there has been an increase in the tendency to perform such multiple dilatations on a patient, either to treat multiple stenoses in a single coronary artery or in several of the coronary arteries.

It has been recognized that it would be desirable to have a single catheter having the capability of performing dilatations with different diameter balloons. In one proposed device, the catheter is provided with two dilatation balloons spaced along the length of the catheter, in tandem, with the smaller of the balloons being located distally of the larger balloon. The tandem balloon arrangement presents several difficulties. Because there is a substantial length of catheter which extends distally of the proximal, larger diameter balloon, the range of locations where the proximal balloon can be placed is limited to the more proximal regions of the coronary artery tree. Additionally, the distal end of the catheter which carries the smaller diameter, distal balloon will be too stiff and will not track properly over the guidewire and through the coronary artery.

Another proposal to provide different diameter dilatation capability on a single catheter has been the use of a single balloon having a stepped configuration in which the distal portion of the balloon is of a smaller diameter than the proximal portion of the balloon, with a transition region joining the two. This configuration presents the difficulty that when the smaller diameter of the portion is placed in a stenosis and inflated that also causes the larger diameter portion of the balloon to inflate. If the artery is too small to accept the larger portion of the inflated balloon, that could result in injury to the patient's artery, possibly rupturing it.

EP-A-0261831 is a conflicting application in accordance with Article 54(3)(4) EPC for the claims for GB, DE, FR, NL and discloses a dilatation catheter for use in angioplasty procedures comprising an elongate catheter body including a main shaft and having a proximal end and a distal end, an inner balloon mounted on the main shaft adjacent the distal end thereof, an outer balloon mounted on the main shaft and enclosing the inner balloon, each of said balloons being formed from a thin, flexible and relatively inelastic material, a first inflation lumen extending through the main shaft having at its distal end an outlet in communication with the interior of the outer balloon, and a second inflation lumen extending through the main shaft and having at its distal end an outlet in communication with the interior of the outer balloon, said first and second inflation lumens being independent of each other to enable the balloons to be inflated or deflated selectively and independently of each.

It is the object of the invention to provide a miltiple balloon dilatation catheter having balloons of different diameters yet which avoids the foregoing and other difficulties.

The present invention according to the claims for GB, DE, FR, NL provides an improvement over EP-A-0261831 (Art.54(3)(4) EPC) in that each of the inner and outer balloons has a proximal end mounted to the main shaft and a distal end mounted to a distal extension connected to the main shaft and means for reinforcing the distal extension from collapsing inwardly under the influence of pressure developed within either of the balloons.

The present invention according to the claims for ES and IT provides an improvement over US-A-4.307.722 in that the balloon mounted on the main shaft is an outer balloon which encloses an inner balloon each of said balloons being formed from a thin, flexible and relatively inelastic material, a first inflation lumen extending through the main shaft having at its distal end an outlet in communication with the interior of the outer balloon and a second inflation lumen extending through the main shaft and having at its distal end an outlet in communication with the interior of the inner balloon, said first and second inflation lumens being indpendent of each other to enable the balloons to be inflated or deflated selectively and independently of each other.

The catheter of the invention includes an elongate catheter shaft having at its distal end a pair of balloons in which one of the balloons is inside of and is completely enclosed within the other, outer balloon. The catheter shaft has two inflation lumens which communicate separately with the balloons. The balloons are inflatable and deflatable independently of each other. The balloons are formed from a very thin, yet strong, relatively inelastic material. The wall thickness of the balloons is sufficiently small so that when the balloons are collapsed the catheter defines a very low profile in the region of the balloons. Thus, although one balloon is contained within the other, the catheter still is capable of being passed through a very narrow stenosis when the balloons are collapsed. The catheter shaft also has a main lumen, open at the distal tip of the catheter, which is receptive to a guidewire and provides fluid communication between the proximal and distal ends of the catheter.

The balloon dilatation catheter of the invention is ideal for use in angioplasty procedures as it has the capability of inflation to a plurality of diameters without risking over-dilatation of other portions of the vessel being treated. Furthermore, it has a very low profile when the balloons are collapsed to enable the balloons to be inserted into a narrow stenosis.

A preferred catheter of the invention will now be described by way of example only, with reference to the accompanying drawings in which:

FIG. 1 is a fragmented plan view of the catheter;

FIG. 2 is a sectional illustration of the main shaft of the catheter as seen along the line 2-2 of FIG.1;

FIG. 3 is an enlarged sectional illustration of the distal end of the catheter as seen along the line 3-3 of FIG. 2 and illustrating the communication of an inflation lumen with the outer balloon;

FIG. 4 is a sectional illustration of the distal region of the catheter as seen along the line 4-4 of FIG. 2 illustrating the communication of an inflation lumen with the inner balloon; and

FIG. 5 is a section of the catheter taken through the balloons as seen along the line 5-5 of FIG. 1 illustrating the balloons in a collapsed, low profile configuration;

FIG. 6 is an illustration similar to FIG. 5 with the inner balloon inflated; and

FIG. 7 is an illustration similar to FIG. 5 in which the outer balloon is inflated.

## DESCRIPTION OF THE PREFERRED EMBODIMENT

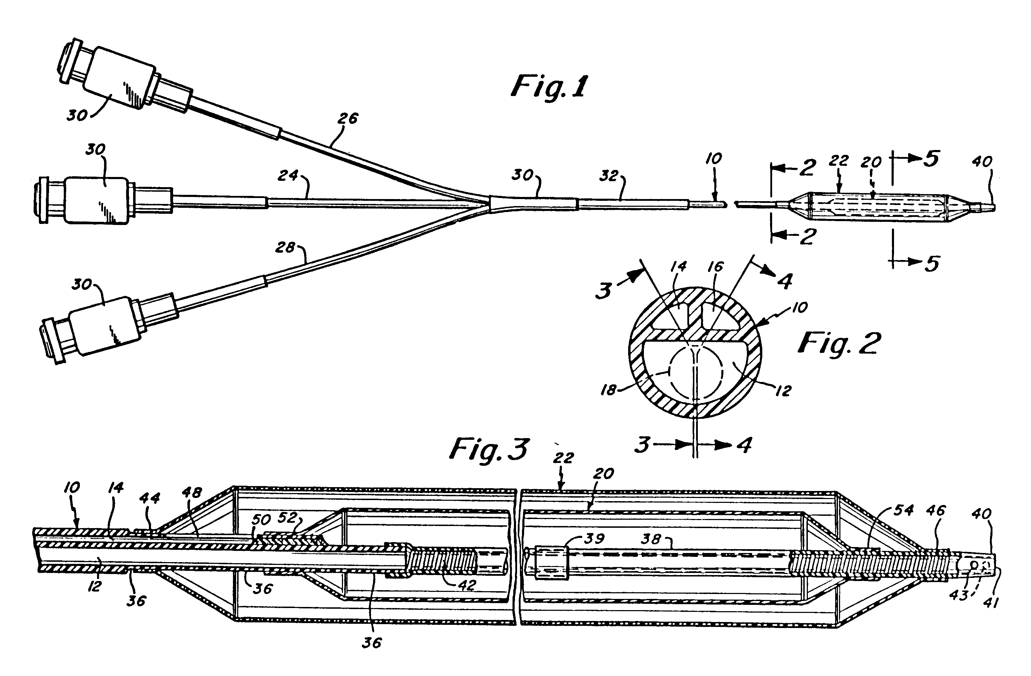

As shown in FIG. 1, the catheter has a catheter body which includes a main shaft 10. The main shaft 10 preferably is formed from an extrusion of a suitably flexible plastic material such as a polyvinyl chloride. The main shaft 10 may have an outer diameter of, for example, 1,5 mm (.058″) and is formed to include a main lumen 12 and a pair of inflation lumens, including a first inflation lumen 14 and a second inflation lumen 16, as shown in FIG. 2. It is preferred to maintain the main lumen 12 as large as is practically possible without making the first and second inflation lumens 14, 16 too small. Additionally, the main lumen 12 should be configured to receive a guidewire (illustrated in phantom at 18) but without fully obstructing the main lumen 12 so as to permit injections of radiopaque dye or the like and pressure measurements to be made as will be appreciated by those familiar with the art. In the preferred embodiment, the main lumen 12 is of a D-shaped configuration and the smaller first and second inflation lumens 14, 16 may be of generally triangular configuration, as shown.

Mounted on the distal end of the catheter are a pair of dilatation balloons, one inside the other and including an inner balloon 20 and an outer balloon 22. The inner balloon 20 has an inflated diameter and length, both of which are smaller than the corresponding dimensions of the outer balloon 22. Each of the inner and outer balloons 20, 22 has a fixed inflated diameter. The inner balloon 20 is in communication with the second inflation lumen 16 and the outer balloon 22 is in communication with the first lumen 14 to enable each of the balloons 20, 22 to be inflated and deflated independently of the other.

The proximal end of the catheter has three tubular legs which may be formed from flexible plastic material including a main leg 24, a first side leg 26 and a second side leg 28. The tubular legs 24, 26, 28 are connected to the catheter main shaft 10 by a junction molding 30. A stress relief tube 32 may be attached to the junction molding 30 and may extend distally over a portion of the main shaft 10 to prevent kinking of the shaft 10 at its juncture with the molding 30. The main leg 24 is aligned within the molding 30 with the main lumen 12. The first and second side legs 26, 28 are in communication with the first and second inflation lumens 14, 16, respectively. The legs 24, 26, 28 are provided with molding 30 at their proximal ends for connection to appropriate fluid devices for inflation and deflation of the balloons or to deliver liquids or the like.

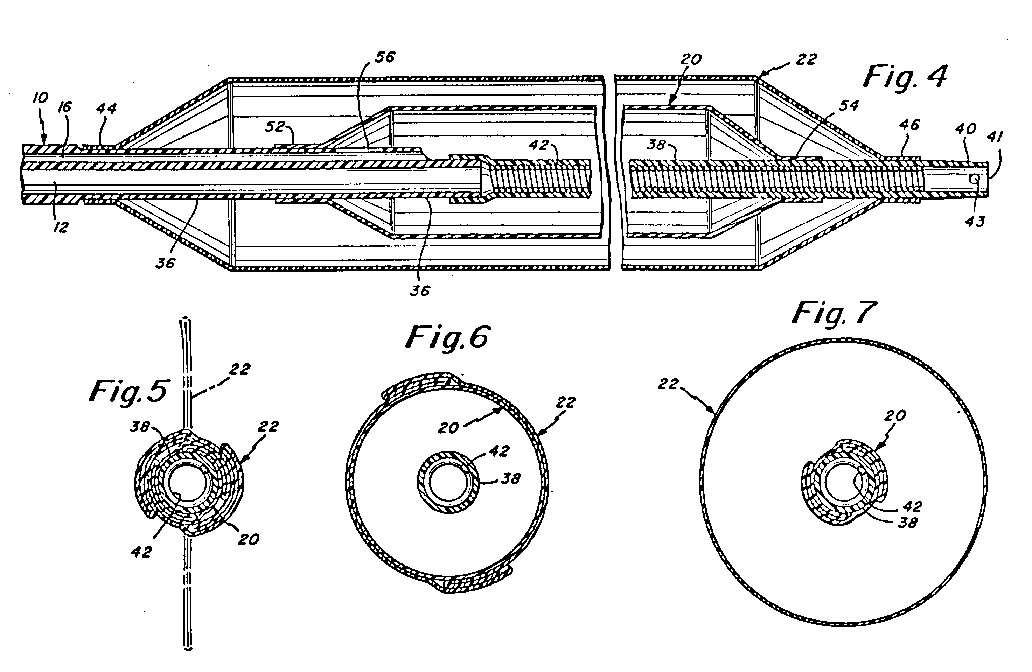

FIGS. 3 and 4 illustrate the distal region of the catheter. The distal end of the main shaft 10 is reduced in diameter to define a smaller diameter shaft distal segment 36. Connected to and about the distal end of the shaft distal segment 36 is a distal extension tube 38 which may be formed from polyvinyl chloride. The distal extension tube 38 extends fully to and defines the distal tip 40 of the catheter. The distal tip 40 includes a distal tip orifice 41 and a pair of side holes 43. The distal extension tube 38 is reinforced internally by a helical spring 42 which extends along and within the distal extension tube 38. The spring 42 reinforces the extension tube 38 to resist collapse of the distal extension tube 38 when the balloons 20, 22 are inflated under high pressures. It may be noted that at the distal portion of the shaft distal segment makes a gradual transition in cross section from the D-shape to a circular shape so as to join smoothly with the distal extension tube 38. The inner diameter of the distal extension tube 38 and reinforcing spring 42 should be large enough to provide clearance about the guidewire 18 sufficiently to permit dye injections and pressure measurements to be made. Preferably, in a diametral clearance of about 0,13-0,15 mm (0.005-0.006″) between the guidewire and lumen of the extension 38 and spring 42 is adequate. The distal extension 38 may be provided with a radiopaque marker band 39 about its mid-region to enhance the visibility of the catheter balloon region under fluoroscopy.

As shown in FIG. 3, the outer balloon 22 in its inflated condition is of substantially uniform diameter along most of its length. The proximal and distal ends of the balloon 22 are formed to define a tapered, conical configuration. The conical portion at the proximal end of the outer balloon 22 terminates in a generally cylindrical collar 44 which is adhesively attached, as by cyanoacrylate adhesive, to the proximal end of the shaft distal segment 36. The distal end of the outer balloon 22 similarly has a distal collar 46 which is adhesively secured to the distal region of the extension tube 38, just proximally of the tip 40. The interior of the outer balloon 22 is maintained in communication with the first inflation lumen 14 by a first port 48 formed in the wall of the shaft distal segment 36. That portion of the first inflation lumen 14 which extends distally beyond the port 48 is filled with adhesive as indicated at 50.

The inner balloon 20 similarly is formed except that it is of a smaller diameter and is shorter than the outer balloon 22. The inner balloon 20 is mounted to the catheter by a proximal collar 52 which is adhesively bonded to the shaft distal segment 36 at a location distal of the port 48. The balloon 20 has a distal collar 54 which is attached to the distal extension tube 38 at a location proximal of the distal collar 46 of the outer balloon 22.

FIG. 4 illustrates the communication of the second inflation lumen 16 with the interior of the second balloon 20. As shown, a second port 56 is formed in the shaft distal segment 36 to communicate the second lumen 16 with the interior of the balloon 20. The end of the second lumen 16 may be left unblocked to provide additional flow area if desired.

By way of illustrative example, the inner balloon may be of the order of 15 mm long and the outer balloon may be of the order of 20 mm long. Preferably the inflated diameters of the balloon differ by about 1 mm. Thus, a catheter may be provided in which the inner balloon has an inflated diameter of 2 mm and an inflated diameter for the outer balloon of 3 mm. Similarly, a catheter having a 3 mm diameter may have an outer balloon of a diameter at least 4 mm. The balloons are formed from very thin, flexible but relatively inelastic material, preferably polyethyelene terephthalate and in a method as described in US-A-4,490,421 to Levy. In order to form the very thin balloon in accordance with the present invention, the starting tubing from which the balloons are made should have a smaller wall thickness than that described in the Levy patent. By way of example, for a 3.0 mm diameter balloon a starting tube of PET material having an inner diameter of 0,43 mm (.0168″) and a wall thickness of 0,11 mm (.0042″) may be stretched as described in the Levy patent to provide balloon double wall thicknesses of 0,008-0,010 mm (0.0003-0.0004 inches). The double wall thickness is a measurement of the thickness of the balloon made by pinching the balloon and measuring the pinched thickness of two layers of the balloon material. Thus, the actual wall thickness of the balloon material is of the order of 0,0038-0,0051 mm (.00015-.0002″). The material is extremely thin and flexible yet provides adequate burst strengths of the order of 12 bars. For polyethylene terephthalate, from which the balloons of the invention are formed, it is preferred to heat the balloon to a temperature of between 110°C to 210°C (with 150°C being preferred) for a period between 10 to 30 seconds until a crystalization reaction has occurred sufficiently to assure the dimensional stability of the balloon.

The extremely thin walls of the balloons permit the balloons to be wrapped about each other when deflated, while providing a very low profile so that the catheter with collapsed balloons can be advanced into very narrow stenoses. By way of example, a catheter so made with a distal extension tube 38 having a diameter of 0,8 mm (.032″) can be passed through an opening about 1 mm (.040″) diameter.

FIG. 5 illustrates, in cross-section, the balloons in a collapsed low profile configuration as they would be when the catheter is inserted into and through a guide catheter. Prior to insertion into the guide catheter, both inner and outer balloons 20, 22 are aspirated through side legs 26, 28 to cause the balloons to collapse. The wings defined by the balloons may be wrapped about the catheter as suggested in FIG. 5 prior to insertion into the guide catheter. It should be noted, however, that the walls of the balloons are very thin and highly flexible so that it is not essential to preliminarily wrap the balloons about the catheter. Simply inserting the catheter with aspirated balloons into the guide catheter is itself sufficient to cause the wings of the balloon to constrict about the catheter. Similarly, when the double balloon catheter is advanced into and through the coronary arteries, it may be passed through narrow constricted regions, such as a narrow stenosis and, in doing so, the wings of the collapsed balloons will wrap about the catheter to permit the double balloon catheter to be inserted into the stenosis.

FIG. 6 illustrates the configuration of the balloons when the smaller diameter inner balloon 20 is inflated. In this configuration, inflation medium is applied only to the second balloon through the second side leg 28 from an appropriate source of fluid pressure. The outer balloon 22 remains uninflated and may be at atmospheric pressure to permit it to wrap closely about the outer diameter of the inflated inner balloon. To the extent that the uninflated outer balloon has excess balloon wall material and defines a wing unable to conform closely to the outer diameter of the inflated inner balloon, that excess material will fold on itself within the stenosis as suggested in FIG. 6.

FIG. 7 illustrates the inflation of the outer balloon. In this configuration, inflation medium is applied only to the outer balloon through the first side leg 26 of an appropriate source of fluid pressure. The inner balloon may be at atmospheric pressure and is permitted to collapse about the extension 38.

In use, the catheter may be advanced together with or over a guidewire into the artery to be treated. The guidewire and catheter may be manipulated to place the balloons within a stenosis to be treated. The physician then may selectively inflate either the inner or the outer balloon depending on the nature of the stenosis. If, after a dilatation has been performed with the smaller diameter inner balloon, there is an apparent need for further dilatation of the stenosis with the larger diameter balloon, there is no need to effect a catheter exchange. The outer balloon is in place within the stenosis and can be inflated immediately to effect the further dilatation. The awkwardness and time delays typically associated with catheter exchanges are avoided. Should the patient have multiple vessel disease or multiple stenoses in the same blood vessel being treated, the catheter can be manipulated and repositioned within the stenoses and the balloon of appropriate diameter may be inflated, again, avoiding the difficulties attendant to a catheter exchange.

For example, a pair of stenoses located in the same coronary artery, one being proximally located and the other being distally located, may be treated in sequence with the invention. The physician may first treat the more proximal stenoses which will be in a larger diameter portion of the coronary artery, by inflating the larger diameter balloon. After that dilatation has been completed and the large balloon deflated, the catheter can be advanced to locate the balloon region within the stenosis in the more distal portion of the artery, where the artery typically will be of a smaller diameter. The dilatation at that stenosis then may be performed by inflating the smaller balloon. Similarly, stenoses in different arteries can be treated by manipulating the catheter, preferably with the assistance of a steerable guidewire, to enable repositioning of the catheter in the desired coronary artery. Thus, the catheter is capable of performing dilatations upon relatively narrow stenoses as well as larger stenoses and in relatively narrow arteries as well as in larger diameter arteries.

From the foregoing, it will be appreciated that the invention provides a multiple balloon catheter by which the necessity for catheter exchanges may be avoided when performing dilatations on a patient having multiple stenoses in one or more arteries. It should be understood, however, that the foregoing description of the invention is intended merely to be illustrative thereof and that other embodiments and modifications may be apparent to those skilled in the art without departing from the scope of the claims.

**Claims**

**Claims for the following Contracting States : ES, IT**

1. A dilatation catheter for use in angioplasty procedures comprising an elongate catheter body including a main shaft (10) having a proximal end and a distal end, and a balloon (20) mounted on the main shaft (10) adjacent the distal end thereof, characterised in that the balloon mounted on the main shaft (10) is an outer balloon (22) which encloses an inner balloon (20) each of said balloons being formed from a thin, flexible and relatively inelastic material, a first inflation lumen (14) extending through the main shaft (10) having at its distal end an outlet (48) in communication with the interior of the outer balloon (22) and a second inflation lumen (16) extending through the main shaft (10) and having at its distal end an outlet (56) in communication with the interior of the inner balloon (20), said first and second inflation lumens (14,16) being independent of each other to enable the balloons to be inflated or deflated selectively and independently of each other.

2. A dilatation catheter as claimed in claim 1 characterised in that said main shaft (10) has a main lumen (18) extending therethrough which terminates at a distal tip orifice (41) distally of the balloons (20,22).

3. A dilatation catheter as claimed in claim 1 or claim 2 characterised in that the balloons (20,22) each have a double wall thickness of about 0.008-0.010mm (0.0003 to 0.0004 inches) and in that the main shaft (10) is less than about 1mm (.040 inches) diameter in the region of the balloons.

4. A balloon dilatation catheter as claimed in claim 3 wherein the balloon region of the catheter, with both balloons deflated, can be passed through a hole 1mm (.040 inches) in diameter.

5. A dilatation catheter as claimed in any preceding claim characterised in that a distal extension (38) is connected to the main shaft (10), each of said inner and outer balloons (20,22) having a proximal end mounted to the main shaft (10) and a distal end mounted to the distal extension (38), and means (42) for reinforcing the distal extension (38) from collapsing inwardly under the influence of pressure developed within either of the balloons (20,22).

6. A dilatation catheter as claimed in any preceding claim characterised in that each of the balloons (20,22) has a burst pressure which is not less than about 12 bars.

**Claims for the following Contracting States : DE, FR, GB, NL**

1. A dilatation catheter for use in angioplasty procedures comprising an elongate catheter body including a main shaft (10) and having a proximal end and a distal end, an inner balloon (20) mounted on the main shaft (10) adjacent the distal end thereof, an outer balloon (22) mounted on the main shaft (10) and enclosing the inner balloon, each of said balloons being formed from a thin, flexible and relatively inelastic material, a first inflation lumen (14) extending through the main shaft (10) having at its distal end an outlet (48) in communication with the interior of the outer balloon (22) and a second inflation lumen (16) extending through the main shaft (10) and having at its distal end an outlet (56) in communication with the interior of the inner balloon (20), said first and second inflation lumens (14,16) being independent of each other to enable the balloons to be inflated or deflated selectively and independently of each other, each of said inner and outer balloons (20,22) having a proximal end mounted to the main shaft (10) and a distal end mounted to a distal extension (38) connected to the main shaft (10) and means (42) for reinforcing the distal extension (38) from collapsing inwardly under the influence of pressure developed within either of the balloons (20,22).

2. A dilatation catheter as claimed in claim 1 characterised in that said main shaft (10) has a main lumen (18) extending therethrough which terminates at a distal tip orifice (41) distally of the balloons (20,22).

3. A dilatation catheter as claimed in claim 1 or claim 2 characterised in that the balloons (20,22) each have a double wall thickness of about 0.008-0.010mm (0.0003 to 0.0004 inches) and in that the main shaft (10) is less than about 1mm (.040 inches) diameter in the region of the balloons.

4. A balloon dilatation catheter as claimed in claim 3 wherein the balloon region of the catheter, with both balloons deflated, can be passed through a hole 1mm (.040 inches) in diameter.

5. A dilatation catheter as claimed in any preceding claim characterised in that each of the balloons (20,22) has a burst pressure which is not less than about 12 bars.

**Patentansprüche**
**Patentansprüche für folgende Vertragsstaaten : ES, IT**

1. Dilatationskatheter zur Verwendung in Angioplastieverfahren mit einem länglichen Katheterkörper mit einem Hauptschaft (10) mit einem proximalen und einem distalen Ende sowie einem Ballon (20) auf dem Hauptschaft (10) nahe dem distalen Ende desselben, dadurch gekennzeichnet, daß der auf dem Hauptschaft (10) sitzende Ballon ein einen inneren Ballon (20) umschließender äußerer Ballon (22) ist, wobei jeder Ballon aus einem dünnen, flexiblen und relativ unelastischem Material hergestellt ist, daß eine erste Aufblaskammer (14) durch den Hauptschaft (10) verläuft und an ihrem distalen Ende einen mit dem Innern des äußeren Ballons (22) in Verbindung stehenden Auslaß (48) aufweist, und daß eine zweite Aufblaskammer (16) durch den Hauptschaft (10) verläuft mit einem an ihrem distalen Ende befindlichen und in Verbindung mit dem Innern des inneren Ballons (20) stehenden Auslaß (56), und daß die erste und die zweite Aufblaskammer (14, 16) unabhängig voneinander sind, damit die Ballons selektiv und jeder für sich aufgeblasen und entleert werden können.

2. Dilatationskatheter nach Anspruch 1, dadurch gekennzeichnet, daß der Hauptschaft (10) eine durch diesen verlaufende Hauptkammer (18) aufweist, die in einem distalen Mundstück (41) distal von den Ballons (20, 22) endet.

3. Dilatationskatheter nach Anspruch 1 oder Anspruch 2, dadurch gekennzeichnet, daß jeder der Ballons (20, 22) eine Doppelwandstärke von ca. 0,008-0,010 mm (0.0003-0.0004") aufweist und daß der Durchmesser des Hauptschafts (10) im Ballonbereich geringer als etwa 1 mm (.040") ist.

4. Dilatationskatheter nach Anspruch 3, dadurch gekennzeichnet, daß mit beiden kollabierten Ballons der Ballonbereich des Katheters durch eine Öffnung von 1 mm (.040") Durchmesser hindurchführbar ist.

5. Dilatationskatheter nach irgendeinem der vorstehenden Ansprüche, dadurch gekennzeichnet, daß eine distale Verlängerung (38) am Hauptschaft (10) angeschlossen ist, wobei sowohl der innere als auch der äußere Ballon (20, 22) jeweils ein mit dem Hauptschaft (10) verbundenes proximales und ein an der distalen Verlängerung (38) sitzendes distales Ende aufweist, und daß Mittel (42) vorgesehen sind, welche die distale Verlängerung (38) gegen ein Kollabieren nach innen unter Einwirkung des in einem der Ballons (20, 22) aufgebauten Drucks abstützen.

6. Dilatationskatheter nach irgendeinem der vorstehenden Ansprüche, dadurch gekennzeichnet, daß jeder Ballon (20, 22) einen Berstdruck von nicht weniger als etwa 12 bar aufweist.

**Patentansprüche für folgende Vertragsstaaten : DE, FR, GB, NL**

1. Dilatationskatheter zur Verwendung in Angioplastieverfahren mit einem länglichen Katheterkörper mit einem Hauptschaft (10) mit einem proximalen und einem distalen Ende, mit einem inneren Ballon (20) auf dem Hauptschaft (10) nahe dem distalen Ende desselben, mit einem auf dem Hauptschaft (10) sitzenden und den inneren Ballon umschließenden äußeren Ballon (22), wobei jeder Ballon aus einem dünnen, flexiblen und relativ unelastischen Material hergestellt ist, mit einer ersten durch den Hauptschaft (10) verlaufenden Aufblaskammer (14), die am distalen Ende einen mit dem Innern des äußeren Ballons (22) in Verbindung stehenden Auslaß (48) aufweist, und mit einer zweiten durch den Hauptschaft (10) verlaufenden Aufblaskammer (16) mit einem an ihrem distalen Ende befindlichen und in Verbindung mit dem Innern des inneren Ballons (20) stehenden Auslaß, wobei die erste und die zweite Aufblaskammer (14, 16) unabhängig voneinander sind, damit die Ballons selektiv und jeder für sich aufgeblasen und entleert werden können, dadurch gekennzeichnet, daß sowohl der innere als auch der äußere Ballon (20, 22) ein am Hauptschaft (10) angeordnetes proximales sowie ein an der distalen Verlängerung (38) des Hauptschafts (10) angeordnetes distales Ende aufweist, und daß Mittel (42) vorgesehen sind, welche die distale Verlängerung (42) gegen ein Kollabieren nach innen unter Einwirkung des in einem der Ballons (20, 22) aufgebauten Drucks abstützen.

2. Dilatationskatheter nach, Anspruch 1, dadurch gekennzeichnet, daß der Hauptschaft (10) eine durch diesen verlaufende Hauptkammer (18) aufweist, die in einem distalen Mundstück (41) distal von den Ballons (20, 22) endet.

3. Dilatationskatheter nach Anspruch 1 oder Anspruch 2, dadurch gekennzeichnet, daß jeder der Ballons (20, 22) eine Doppelwandstärke von ca. 0,008-0,010 mm (0.0003-0.0004") aufweist und daß der Durchmesser des Hauptschafts (10) im Ballonbereich geringer als etwa 1 mm (.040") ist.

4. Dilatationskatheter nach Anspruch 3, dadurch gekennzeichnet, daß mit beiden kollabierten Ballons der Ballonbereich des Katheters durch eine Öffnung von 1 mm (.040") Durchmesser hindurchführbar ist.

5. Dilatationskatheter nach irgendeinem der vorstehenden Ansprüche, dadurch gekennzeichnet, daß jeder Ballon (20, 22) einen Berstdruck von nicht weniger als etwa 12 bar aufweist.

**Revendications**
**Revendications pour les Etats contractants suivants : ES, IT**

1. Cathéter à dilatation destiné aux interventions d'angioplastie comportant un cathéter à corps allongé prévoyant une tige principale (10) ayant une extrémité proximale et une extrémité distale, et un sac intérieur (20) monté sur la tige principale (10) à proximité de l'extrémité distale, **caractérisé en ce que** le sac monté sur la tige principale (10) est un sac extérieur (22) autour d'un sac intérieur (20), chacun desdits sacs étant formé à partir de matière fine, souple et relativement non-élastique, un premier passage de gonflement (14) allant de part et d'autre de la tige principale (10), ayant à son extrémité distale une sortie (48) communiquant avec l'intérieur du sac extérieur (22) et un deuxième passage de gonflement (16) allant de part et d'autre de la tige principale (10) et ayant à son extrémité distale une sortie (56) communiquant avec l'intérieur du sac intérieur (20), lesdits premier et deuxième passages de gonflement (14,16) étant indépendant l'un de l'autre de façon à en permettre le gonflement ou le dégonflement sélectif et indépendant l'un de l'autre.

2. Cathéter à dilatation selon la revendication 1, **caractérisé en ce que** ladite tige principale (10) prévoit un passage principal (18) le traversant de part et d'autre et se terminant en orifice d'extrémité distale (41) et en rapport distal avec les sacs (20,22).

3. Cathéter à dilatation selon la revendication 1 ou la revendication 2, **caractérisé en ce que** les sacs (20,22) sont chacun d'une épaisseur de double paroi d'environ 0,008-0,010mm (0,0003 0,0004") et en ce que le diamètre de la tige principale (10) est inférieure à environ 1,0mm (0,040") à proximité des sacs.

4. Cathéter à dilatation selon la revendication 3 selon laquelle, lorsque les deux sacs sont dégonflés, la zone du sac du cathéter est en mesure de passer par un trou mesurant 1,0mm (0,040") de diamètre.

5. Cathéter à dilatation selon l'une ou l'autre des revendications précédentes, **caractérisé en ce qu'**une rallonge distale (38) est raccordée à la tige principale (10), chacun desdits sacs intérieur et extérieur (20,22) ayant une extrémité proximale montée sur la tige principale (10) et une extrémité distale montée sur la rallonge distale (42), et des moyens 42) de renfort de la rallonge distale (38) évitant l'affaissement intérieur sous la pression engendrée dans l'un ou l'autre des sacs (20,22).

6. Cathéter à dilatation selon l'une ou l'autre des revendications précédentes, **caractérisé en ce que** la pression d'éclatement de chacun des sacs (20,22) n'est pas inférieure à 12 bars.

**Revendications pour les Etats contractants suivants : DE, FR, GB, NL**

1. Cathéter à dilatation utilisable dans les procédures d'angioplastie, comprenant un corps de cathéter allongé comportant un arbre principal (10) et ayant une extrémité proximale et une extrémité distale, un ballon intérieur (20) monté sur l'arbre principal (10) au voisinage de son extrémité distale, un ballon extérieur (22) monté sur l'arbre principal (10) et renfermant le ballon intérieur, chacun de ces ballons étant formé d'un matériau mince, souple et relativement non élastique, un premier passage de gonflage (14) s'étendant à travers l'arbre principal (10) ayant à son extrémité distale une lumière (48) en communication avec l'intérieur du ballon extérieur (22) et un second passage de gonflage (16) s'étendant à travers l'arbre principal (10) et ayant à son extrémité distale une lumière (56) en communication avec l'intérieur du ballon intérieur (20), ces passages de gonflage (14, 16) étant indépendants l'un de l'autre pour permettre aux ballons d'être gonflés et dégonflés sélectivement et indépendamment l'un de l'autre, chacun des ballons (20, 22) ayant une extrémité proximale montée sur l'arbre principal (10) et une extrémité distale montée sur un prolongement distal (38) relié à l'arbre principal (10) et des moyens (42) pour le renforcement du prolongement distal (38) pour l'empêcher de s'écraser vers l'intérieur sous l'influence de la pression développée par l'un ou l'autre des ballons (20, 22).

2. Cathéter à dilatation selon la revendication 1, caractérisé en ce que ledit arbre principal (10) comporte un passage principal (18) le traversant de bout en bout et se terminant par un orifice distal (41) situé distalement par rapport aux ballons (20, 22).

3. Cathéter à dilatation selon la revendication 1 ou la revendication 2 caractérisé en ce que les ballons (20, 22) ont chacun une épaisseur de double paroi d'environ 0,008 - 0,010 mm et en ce que l'arbre principal (10) a un diamètre inférieur à environ 1 mm dans la région des ballons.

4. Cathéter à dilatation de ballon selon la revendication 3, dans lequel la région des ballons du cathéter - les deux ballons étant dégonflés - peut passer à travers un trou de 1 mm de diamètre.

5. Cathéter à dilatation selon l'une quelconque des revendications précédentes, caractérisé en ce que chacun des ballons (20, 22) a une pression d'éclatement non inférieure à 12 bars.

Fig.1

Fig.2

Fig.3

Fig.5

Fig.6

Fig.7

Fig.4